# EUROPEAN PATENT SPECIFICATION

(11) **EP 2 886 037 B1**
(45) Date of publication and mention of the grant of the patent: **23.05.2018**
(21) Application number: 14196798.4
(22) Date of filing: 08.12.2014
(51) Int. Cl.: A61B 1/00, A61B 1/12

(54) **ENDOSCOPE WITH NOZZLE**
ENDOSKOP MIT DÜSE
ENDOSCOPE AVEC BUSE

(30) Priority: 20.12.2013 JP 2013263740
(43) Date of publication of application: 24.06.2015
(73) Proprietor: FUJIFILM Corporation, Tokyo 106-8620 (JP)
(72) Inventor: Ikeda, Toshiyuki, Ashigarakami-gun Kanagawa (JP); Torii, Yuichi, Ashigarakami-gun Kanagawa (JP); Iyama, Shozo, Ashigarakami-gun Kanagawa (JP)
(74) Representative: Klunker IP Patentanwälte PartG mbB

(56) References cited:
- EP-A1- 2 494 909
- EP-A1- 2 859 835
- US-A1- 2011 112 363

## Description

### BACKGROUND OF THE INVENTION

### 1. Field of the Invention

The present invention relates to an endoscope according to the preamble of claim 1. More particularly, the present invention relates to an endoscope with a nozzle, in which residual presence of droplets of water can be prevented in a lighting window area at a distal end.

### 2. Description Related to the Prior Art

An endoscope includes an elongated tube and a tip device disposed at a distal end of the elongated tube for entry in a body cavity. The tip device includes lighting window areas, a viewing window area and a nozzle device. The lighting window areas emit light for illuminating an object of interest in the body cavity. The viewing window area receives image light from the obj ect. The nozzle device ejects fluid to the viewing window area, such as water and gas (for example, air or carbon dioxide gas) . A nozzle opening of the nozzle device ejects water to a surface of the viewing window area to wash away body fluid, waste fluid or the like. Then the nozzle opening ejects air to blow away droplets of the water remaining on the viewing window area. Should the waste fluid or the like remain locally on the viewing window area, a problem arises in the quality of imaging. The fluid from the nozzle device is preferably spread to the entire window surface of the viewing window area.

Improvements of the tip device of the elongated tube are disclosed in US 2011 112363 A1 and US 2014 039260 A1 (corresponding to JP-A 2011-120863), and US 8,777,845 B1 and US 2014 058204 A1 (corresponding to JP-A 2012-179221). Said US 2011 112363 A1 discloses an endoscope according to the preamble of claim 1. The inclined guide surface extends around a portion of the viewing window opposite to the nozzle device. A window surface of the viewing window area is disposed at a higher level than a flat end surface of the tip device. An inclined portion is formed along a peripheral edge of the viewing window area and has a height gradually increasing from the end surface toward the window surface. The fluid ejected by the nozzle opening of the nozzle device impinges upon the inclined surface of the inclined portion, and flows smoothly toward the window surface. The fluid can be spread to the entire window surface of the viewing window area.

According to US 2011 112363 A1, US 2014 039260 A1, US 8,777,845 B1 and US 2014 058204 A1, the fluid from the nozzle device flows at a higher flow rate in a central portion of the nozzle opening, and flows at a lower flow rate in peripheral portions of the nozzle opening. As the nozzle device is used for selectively ejecting both of the water and the gas, an ejection path of those may be different according to differences of their specifics, such as viscosity, relative weight and the like. The ejection path of the gas is laterally larger than the ejection path of the water. In a normal use of the endoscope, the nozzle device ejects the water for washing the viewing window area before the nozzle device ejects the gas for blowing away the droplets from the viewing window area. In view of this, the nozzle device is so constructed that a flow stream of the gas through the central portion of the nozzle device can reliably blow away the water by use of the higher flow rate. However, a problem occurs with a flow stream of the gas through the peripheral portions of the nozzle opening. The water on the end surface as the droplets is moved, and comes to and remains on the lighting window areas. The residual presence of the droplets is remarkable specifically with the largeness in a distance from the nozzle opening of the nozzle device.

The residual presence of the droplets on the lighting window areas may degrade endoscopic images due to halation or flare with the droplets. Also, the droplets may be imaged in the view field to narrow an available area of view, typically in a wide-angle type of the endoscope with the view field which has been recently used widely. The viewing window area is preferably disposed separately from the lighting window areas for the purpose of reducing residual presence of the droplets on the end surface in the tip device. The droplets are guided to the outside of the end surface by passing between the viewing window area and the lighting window areas. The droplets may come to the outside of the view field on the end surface, and will not influence to the quality of the imaging. However, there is important requirement of reducing the diameter of the elongated tube with the tip device for the purpose of reducing physical stress to a body of a patient. Also, in contrast with this, there is a conception of enlarging the diameter of the viewing window area and the lighting window areas for the purpose of high image quality in endoscopic imaging. However, there is no known structure in which the viewing window area and the lighting window areas are disposed near to one another and in which residual presence of the droplets around the lighting window areas is prevented.

### SUMMARY OF THE INVENTION

In view of the foregoing problems, an object of the present invention is to provide an endoscope with a nozzle, in which residual presence of droplets of water can be prevented in a lighting window area at a distal end.

In order to achieve the above and other objects and advantages of this invention, an endoscope having an elongated tube for entry in a body cavity includes the features of claim 1.

Preferably, the nozzle device selectively ejects liquid and gas. The ejection path of the gas is laterally larger than the ejection path of the liquid. The viewing window unit is disposed inside the ejection path of the liquid, and the lighting window unit is disposed inside the ejection path of the gas.

Preferably, the lighting window unit is constituted by first and second lighting window units. The inclined guide surface is disposed between the first lighting window unit and the nozzle device.

Preferably, the first lighting window unit is disposed farther from the nozzle device than the second lighting window unit, and nearer to the suction opening than the second lighting window unit.

Preferably, the inclined guide surface is disposed between the first and second lighting window units.

Preferably, the tip device includes a head shell and an end cap mounted on a distal end of the head shell. The inclined guide surface is formed with the end cap.

Preferably, the suction opening is disposed close to the nozzle device.

Preferably, the lighting window unit is disposed close to the tapered surface.

Preferably, the suction opening is a distal channel opening for an instrument.

Preferably, the viewing window unit protrudes from the end surface at a height equal to or more than 0.1 mm and equal to or less than 0.3 mm.

Preferably, the lighting window unit is constituted by first and second lighting window units. The suction opening is disposed between the nozzle device and the first lighting window unit, and the nozzle device is disposed between the second lighting window unit and the suction opening.

Preferably, the nozzle device includes a nozzle sleeve, incorporated in the tip device, for flow of the fluid in the axial direction. A nozzle opening is formed in a sleeve end of the nozzle sleeve to open in a direction crosswise to the axial direction, for ejecting the fluid from the nozzle sleeve on the end surface.

Preferably, furthermore, a liquid conduit is provided through the elongated tube, for supplying the nozzle device with liquid. A gas conduit is provided through the elongated tube, for supplying the nozzle device with gas. A valve device changes over the liquid conduit and the gas conduit, so as to change over the liquid and the gas to be ejected through the nozzle device.

Consequently, residual presence of droplets of water can be prevented in a lighting window area at a distal end, because the inclined guide surface guides the water.

### BRIEF DESCRIPTION OF THE DRAWINGS

The above objects and advantages of the present invention will become more apparent from the following detailed description when read in connection with the accompanying drawings, in which:
Fig. 1 is an explanatory view illustrating an endoscope system;
Fig. 2 is a vertical section illustrating an endoscope having plural channels;
Fig. 3 is a perspective view illustrating a tip device of the endoscope;
Fig. 4 is a vertical section taken on line IV-IV in Fig. 3, illustrating the tip device;
Fig. 5 is a plan illustrating the tip device where fluid is ejected to a viewing window unit;
Fig. 6 is a plan illustrating the tip device where droplets are guided.

### DETAILED DESCRIPTION OF THE PREFERRED

### EMBODIMENT(S) OF THE PRESENT INVENTION

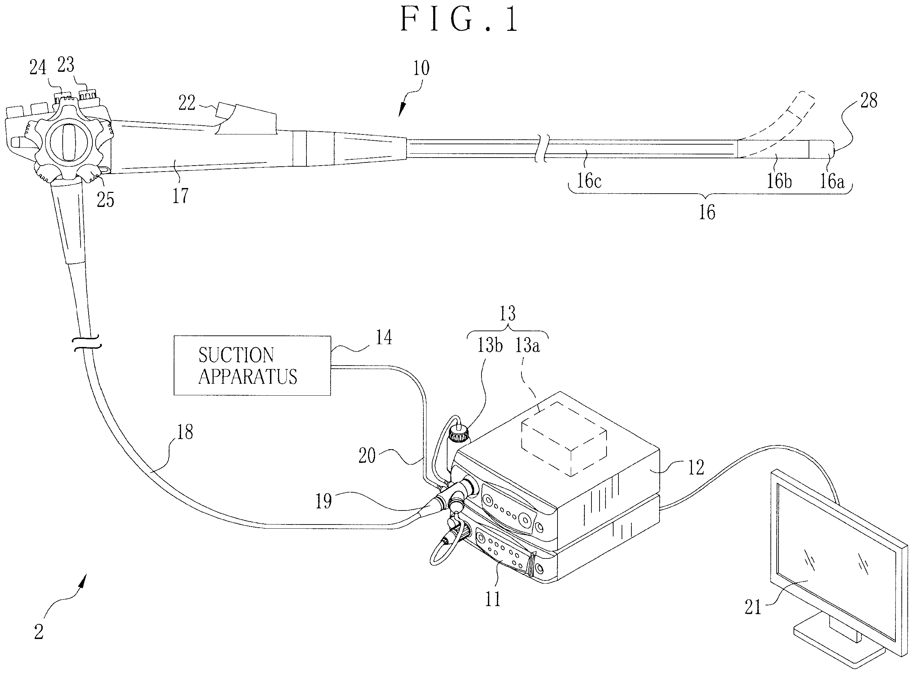

In Fig. 1, an endoscope system 2 includes an electronic endoscope 10, a processing apparatus 11, a light source apparatus 12, a fluid supply apparatus 13 and a negative pressure source 14 or suction apparatus. The fluid supply apparatus 13 includes a gas supply source 13a with a pump, and a water supply source 13b with a tank. The gas supply source 13a is incorporated in the light source apparatus 12, and supplies gas. The water supply source 13b is disposed outside the light source apparatus 12, and stores washing water. The endoscope 10 includes an elongated tube 16, a grip handle 17 and a universal cable 18. The elongated tube 16 is entered in a body cavity. The grip handle 17 is disposed at a proximal end of the elongated tube 16. The universal cable 18 is connected with the processing apparatus 11 and the light source apparatus 12.

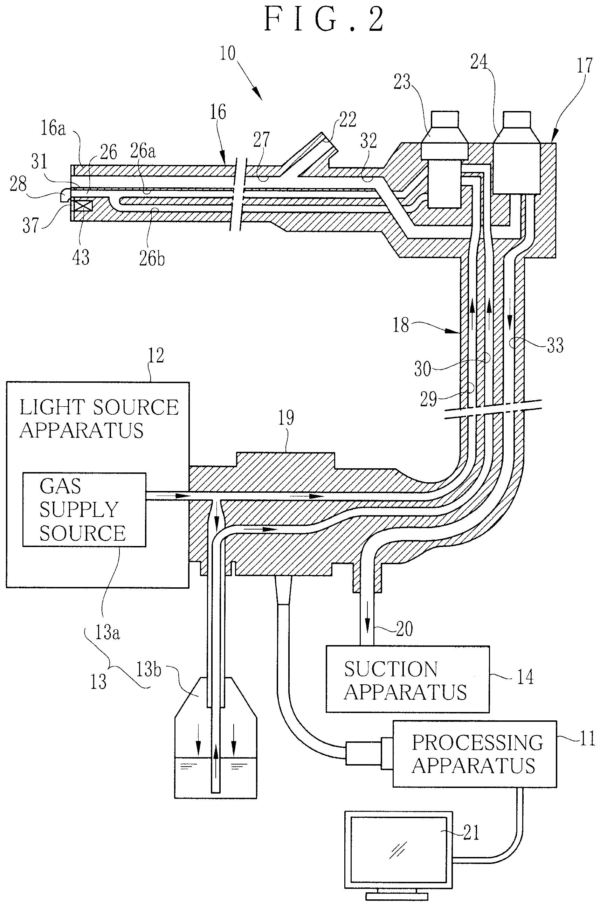

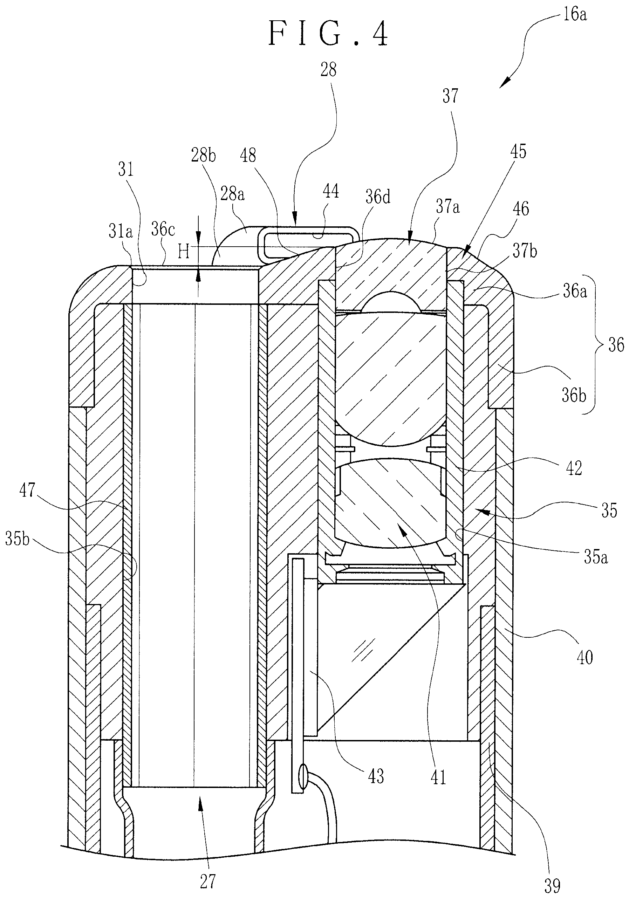

The elongated tube 16 includes a tip device 16a, a steering device 16b and a flexible tube device 16c. A CCD image sensor 43 is incorporated in the tip device 16a for imaging in a body cavity. See Figs. 2 and 4. The steering device 16b is disposed on a proximal end of the tip device 16a. The flexible tube device 16c is disposed to extend from the steering device 16b. Expressions of proximal and distal sides are used according to the orientation of the elongated tube 16.

A connection plug 19 is disposed at a proximal end of the universal cable 18. The connection plug 19 is a composite type of connector for connection to the processing apparatus 11, the light source apparatus 12 and the fluid supply apparatus 13. A flow tube 20 is disposed to couple the connection plug 19 to the negative pressure source 14.

The processing apparatus 11 is electrically connected to the light source apparatus 12, and controls circuit elements in the endoscope system 2. A cable line is disposed to extend through the universal cable 18 and the elongated tube 16, and used by the processing apparatus 11 to supply the endoscope 10 with power, and to control operation of the image sensor 43. Also, an image signal output by the image sensor 43 is received by the processing apparatus 11 through the cable line, and generates image data by performing image processing of various functions. According to the image data formed by the processing apparatus 11, a monitor display panel 21 in connection with the processing apparatus 11 by a cable line is driven to display an image.

The grip handle 17 has a proximal channel opening 22, a fluid supply button 23 (valve device), a suction button 24 and steering wheels 25. In case the steering wheels 25 are rotated, control wires through the elongated tube 16 are moved back or forth to bend the steering device 16b up or down or to the right or left. Thus, the tip device 16a is directed in a desired direction in the body cavity.

In Fig. 2, there are a flow channel 26 and an instrument channel 27 formed through the elongated tube 16 and the grip handle 17. A nozzle device 28 for water and gas is disposed with the tip device 16a. A distal end of the flow channel 26 communicates with the nozzle device 28. A gas conduit 26a and a water conduit 26b (liquid conduit) are disposed to extend from a proximal end of the flow channel 26. The gas conduit 26a and the water conduit 26b are connected to the fluid supply button 23 in the grip handle 17.

A gas supply conduit 29 is disposed to communicate with the gas supply source 13a. A water supply conduit 30 is disposed to communicate with the water supply source 13b. The gas supply conduit 29 and the water supply conduit 30 are connected to the fluid supply button 23 as well as the gas conduit 26a and the water conduit 26b. The gas supply source 13a supplies gas, such as air or carbon dioxide gas, at the time of imaging with the endoscope 10.

In case the fluid supply button 23 is depressed for gas supply, gas from the gas supply source 13a is drawn to flow to the nozzle device 28. In case the fluid supply button 23 is depressed for water supply, water from the water supply source 13b is drawn to flow to the nozzle device 28 in a condition with pressure of the gas from the gas supply source 13a. The nozzle device 28 selectively ejects gas or water supplied through the flow channel 26.

A distal channel opening 31 or suction opening is a distal end of the instrument channel 27. The proximal channel opening 22 is a proximal end of the instrument channel 27. One of various medical instruments, such as an injection needle, high frequency knife and the like, is entered into the proximal channel opening 22. In a normal state of not using the medical instruments, a closing cap (not shown) closes the proximal channel opening 22. A suction conduit 32 is a branch of the instrument channel 27, and extends to the suction button 24.

An exhaust conduit 33 has first and second ends. The first end is coupled to the negative pressure source 14 by the flow tube 20. The second end is coupled to the suction button 24 in addition to the suction conduit 32. During the imaging of the endoscope 10, the negative pressure source 14 is active constantly. In case the suction button 24 is depressed for suction, negative pressure is applied by the negative pressure source 14. In case the suction button 24 is manipulated for stop, no negative pressure is applied, so that the suction is interrupted.

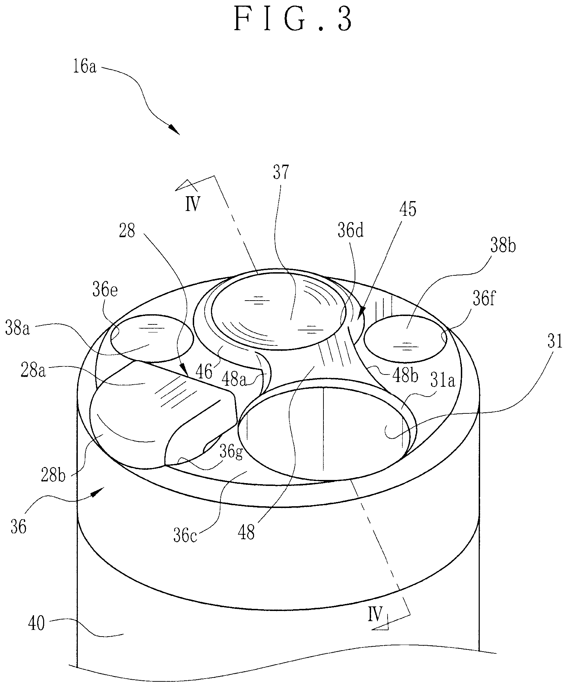

In Figs. 3, 4 and 5, the tip device 16a includes a head shell 35, an end cap 36, a viewing window unit 37 or viewing window area, lighting window units 38a and 38b or lighting window areas, the nozzle device 28 and the distal channel opening 31. The end cap 36 is fitted on the head shell 35. Channel holes 35a and 35b are formed through the head shell 35 in an axial direction of the elongated tube 16, and hold various elements including the nozzle device 28, an objective lens 41 or objective lens optics, a flow sleeve 47 and a light guide device. A distal link element 39 included in plural link elements arranged in series in the steering device 16b is connected with a proximal end of the head shell 35. In Fig. 4, a thickness of each of the various elements in the radial direction is depicted for clarification in an emphasized form in comparison with Fig. 3. The distal channel opening 31 and the viewing window unit 37 have actual diameters larger than those in Fig. 4.

The end cap 36 includes a cap plate 36a and a cap skirt 36b. The cap plate 36a covers a distal end of the head shell 35. The cap skirt 36b is fitted on the periphery of the head shell 35. A jacket layer 40 is disposed in the outside of the steering device 16b, and extends to the head shell 35. A distal end of the jacket layer 40 is fitted on a proximal end of the cap skirt 36b, and firmly attached to the same by adhesive agent or the like. A flat end surface 36c of the cap plate 36a is a plane perpendicular to the axial direction of the elongated tube 16 at a distal end of the elongated tube 16. Note that the perpendicular direction of the cap plate 36a includes a perpendicular direction and a nearly perpendicular direction.

Mount openings 36d, 36e, 36f and 36g are formed in the cap plate 36a as well as the distal channel opening 31. The viewing window unit 37 and the lighting window units 38a and 38b are disposed inside respectively the mount openings 36d-36f. The nozzle device 28 is disposed inside the mount opening 36g.

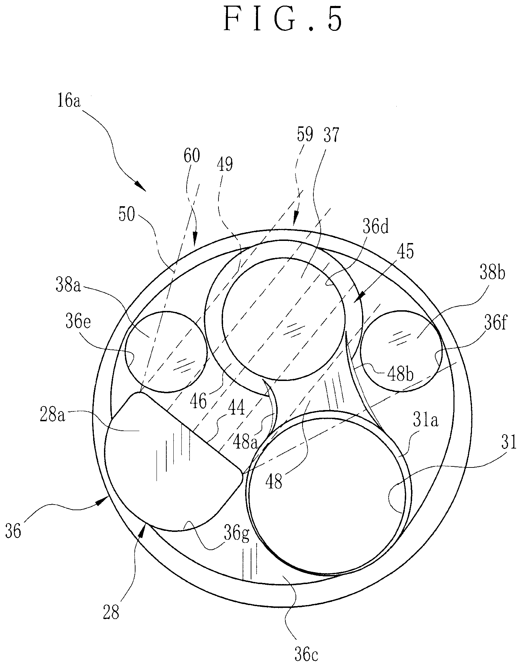

Note that the upper and lower sides in Fig. 5 are equal to those of orientation of the steering device 16b in the steering. The viewing window unit 37 is disposed on the upper side in the end surface 36c. The nozzle device 28 and the distal channel opening 31 are disposed lower than the viewing window unit 37. The lighting window units 38a and 38b are arranged symmetrically with one another with respect to the viewing window unit 37.

The viewing window unit 37 is a front lens component included in the objective lens 41, and operates also as a glass cover. An outer form of the viewing window unit 37 is circular. A window surface 37a as an incident surface is convex or flat. In the present embodiment, the window surface 37a is convex. A lens barrel 42 contains optics of the objective lens 41 including the viewing window unit 37. A proximal portion of a peripheral surface 37b of the viewing window unit 37 is fitted in the lens barrel 42. A distal portion of the peripheral surface 37b is fitted in the mount opening 36d of the end cap 36.

The lens barrel 42 is fitted in the channel hole 35a of the head shell 35. A distal end surface of the lens barrel 42 is fitted on the cap plate 36a of the end cap 36, so that the viewing window unit 37 protrudes from the end surface 36c. Thus, the window surface 37a of the viewing window unit 37 is disposed higher than the end surface 36c. A height of the protrusion of the window surface 37a of the viewing window unit 37 from the end surface 36c is 0.1-0.3 mm.

Note that the viewing window unit 37 can be a glass cover of a flat shape without a lens structure at the distal end of the objective lens 41. Also, the objective lens 41 may not be provided in the viewing window unit 37. Only a glass cover can constitute the viewing window unit 37 and can be fitted in the mount opening 36d in the end cap 36.

In Fig. 4, the image sensor 43 is disposed behind the objective lens 41. An example of the image sensor 43 is an interline transfer type of CCD. An object image is formed optically by the objective lens 41 and focused on the image sensor 43. Note that other examples of the image sensor 43 can be used, such as CMOS and the like.

The lighting window units 38a and 38b have a structure of a lighting lens, and apply light from the light source apparatus 12 to an object of interest in a body cavity of a patient. A light guide device (not shown) is disposed and has an exit surface positioned at each of the lighting window units 38a and 38b. The light guide device is constituted by a plurality of optical fibers, extends through the elongated tube 16, the grip handle 17, the universal cable 18 and the connection plug 19, and guides light from the light source apparatus 12 to the lighting window units 38a and 38b. Note that an example of the light from the light source apparatus 12 can be excitation light of laser light. Preferably, a lighting system can include fiber optics of a single filament and phosphor. The fiber optics guide excitation light from the light source apparatus 12. The phosphor is disposed at the tip device 16a, and receives the excitation light to generate light for illumination.

A nozzle sleeve 28b at a proximal end of the nozzle device 28 is fitted on an outer surface of a sleeve of the flow channel 26 for coupling. The nozzle sleeve 28b of the nozzle device 28 and the flow channel 26 are fitted in the mount opening 36g of the head shell 35. Also, the nozzle device 28 includes a nozzle spout 28a and a nozzle opening 44. The nozzle spout 28a is directed from the nozzle sleeve 28b in an L-shape. The nozzle opening 44 is open at a tip end of the nozzle spout 28a. On the end surface 36c, the nozzle spout 28a is positioned, as the nozzle sleeve 28b is fitted in the mount opening 36g of the end cap 36.

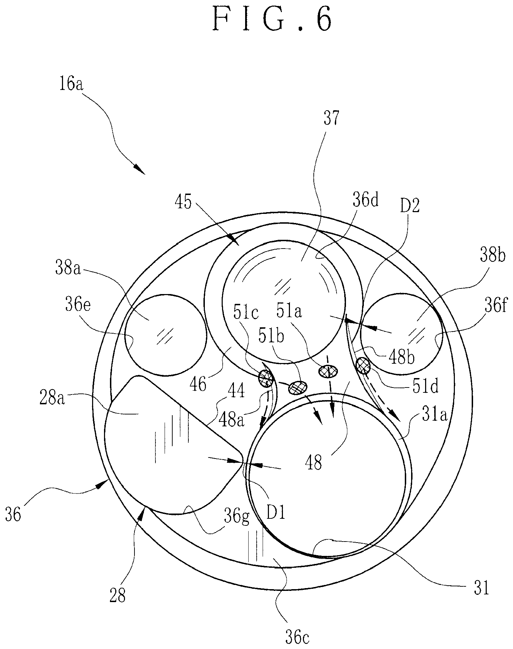

The nozzle device 28 is disposed near to the distal channel opening 31. Note that the nearness of the nozzle device 28 to the distal channel opening 31 means a state with a distance D1 (Fig. 6) equal to or more than 0 mm and equal to or less than 0.5 mm.

The nozzle device 28 selectively ejects washing water 49 (liquid) and air 50 (gas). In Fig. 5, the washing water 49 is indicated by the dotted line, and ejected by the nozzle opening 44 toward the viewing window unit 37 substantially in a linear manner. The ejected air 50 is indicated by the phantom line, and ejected in a manner of increasing a path width according to the distance form the nozzle device 28. The viewing window unit 37 is disposed inside a first ejection path 59 of the washing water 49 ejected by the nozzle device 28. In the nozzle device 28, a flow component of the fluid (the washing water 49 or the ejected air 50) flowing out through the center of the nozzle opening 44 flows at a high flow rate. A flow component of the fluid flowing out through lateral ends of the nozzle opening 44 flows at a lower flow rate.

The lighting window units 38a and 38b are disposed beside the viewing window unit 37, and flush with the end surface 36c. A second ejection path 60 of gas is defined downstream of the nozzle device 28. The lighting window units 38a and 38b are disposed inside the second ejection path 60. The lighting window unit 38b is nearer to the distal channel opening 31 than the viewing window unit 37. Also, the lighting window unit 38b is distant from the nozzle device 28. The lighting window unit 38a is farther from the distal channel opening 31 than the viewing window unit 37. Also, the lighting window unit 38a is near to the nozzle device 28. There is a tapered surface 46 to which the lighting window units 38a and 38b are arranged near. Note that the nearness of the lighting window units 38a and 38b to the tapered surface 46 means a state with a distance D2 (Fig. 6) equal to or more than 0.1 mm and equal to or less than 0.3 mm.

The flow sleeve 47 for connection is fitted in the channel hole 35b of the head shell 35, and couples the distal channel opening 31 to the instrument channel 27 to communicate with the proximal channel opening 22. A medical instrument entered into the proximal channel opening 22 is caused to protrude through the distal channel opening 31 distally. The distal channel opening 31 operates as a suction opening for suction with negative pressure through the path with the instrument channel 27 in connection with the suction conduit 32. The distal channel opening 31 sucks and removes various fluids, such as washing water, residual body fluid and the like.

An annular projection 45 is formed with the end cap 36 and between a peripheral edge of the viewing window unit 37 and the end surface 36c, and projects from the end surface 36c at a predetermined height. An inner surface of the annular projection 45 extends continuously from the inside of the mount opening 36d. The tapered surface 46 is an outer surface of the annular projection 45. The tapered surface 46 has a diameter decreasing from the end surface 36c toward the viewing window unit 37 and is disposed around the viewing window unit 37. A height H of the annular projection 45 projecting from the end surface 36c is 0.05-0.3 mm.

An inclined guide surface 48 (ramp surface) is formed with the tapered surface 46, between the lighting window unit 38b and the nozzle device 28, and on a straight line passing centers of the viewing window unit 37 and the distal channel opening 31. The inclined guide surface 48 is defined by extending a portion of the tapered surface 46 toward the distal channel opening 31, to form a wall from the viewing window unit 37 to the distal channel opening 31. The inclined guide surface 48 and the annular projection 45 have the same height H on the periphery of the viewing window unit 37.

The inclined guide surface 48 is disposed inside the second ejection path 60 of the gas ejected by the nozzle device 28, and inclined linearly from the periphery of the viewing window unit 37 toward the distal channel opening 31. Note that the expression of the linear form includes an exactly linear form and a nearly linear form. Furthermore, the tapered surface 46 or the inclined guide surface 48 may be a curved surface continuing to the window surface 37a of the viewing window unit 37.

There are a concave side surface 48a and a side surface 48b beside the inclined guide surface 48. The concave side surface 48a is opposed to the nozzle device 28, and concave with a curve in a direction away from the nozzle device 28. Preferably, the concave side surface 48a is inclined in a tapered manner with respect to the end surface 36c. The side surface 48b on the side reverse to the concave side surface 48a is curved concavely in a direction opposite to the concave side surface 48a. An inclined inner surface 31a is formed with an edge of the distal channel opening 31 continuing from the inclined guide surface 48, for preventing interference of a tip of a medical instrument.

A process of washing the viewing window unit 37 by ejecting fluid from the nozzle device 28 is described now. The viewing window unit 37 and the tapered surface 46 are disposed inside the ejection path 59 or 60 of the nozzle device 28. Part of washing water from the nozzle device 28 directly impinges upon the viewing window unit 37. The remainder of the washing water comes in contact with the tapered surface 46, becomes spread in the circumferential direction of the viewing window unit 37, and comes upon on the tapered surface 46. Thus, the washing water comes to flow to the entire surface of the viewing window unit 37, to blow off or remove body liquid or other unwanted fluid from the viewing window unit 37. Also, ejection of gas blows off the residual washing water from the viewing window unit 37.

Specifically, washing water ejected through peripheral portions of the nozzle opening 44 flows at a low flow rate among flow streams from the nozzle device 28. A droplet is likely to remain on the end surface 36c near the nozzle device 28 at the end of the ejection of the washing water. Also, residual washing water near to the nozzle device 28 after the ejection of water or before ejection of gas is likely to remain on the end surface 36c of the inclined guide surface 48 (ramp surface) as droplets. It is possible reliably to blow away part of droplets in a center area of a high flow rate of a flow stream among flow streams of gas from the nozzle device 28. However, part of the droplets in an area of a low flow rate is likely to move toward the lighting window unit 38b and remain again without being blown away.

However, the inclined guide surface 48 is disposed within the second ejection path 60 of gas from the nozzle device 28, between the lighting window unit 38b and the nozzle device 28, and on a straight line passing the centers of the viewing window unit 37 and the distal channel opening 31 (suction opening) . In Fig. 6, a residual droplet 51a and a droplet 51b moved from the end surface 36c are caused to flow along the inclined guide surface 48. A residual droplet 51c on the end surface 36c nearer to the nozzle device 28 than the inclined guide surface 48 also flows along the concave side surface 48a beside the inclined guide surface 48 toward the distal channel opening 31. A droplet 51d having passed the inclined guide surface 48 flows along the side surface 48b to the distal channel opening 31 or to the periphery of the end cap 36.

Also, manipulation of the suction button 24 can introduce a droplet to the distal channel opening 31 from the inclined guide surface 48 or near the lighting window unit 38b, and removed by suction. The lighting window unit 38a is nearer to the nozzle device 28 than the lighting window unit 38b. Residual droplets near to the lighting window unit 38a are disposed in an area of a flow stream with a high flow rate within the ejection path of gas ejected by the nozzle device 28. Thus, the droplets are blown away by the gas from the nozzle device 28.

Thus, it is possible reliably to remove residual and moving droplets by forming the inclined guide surface 48 with the tip device 16a. Halation and flare can be prevented to keep high quality of endoscopic images without degradation. Reach of droplets to the lighting window unit 38b is prevented by the inclined guide surface 48 between the nozzle device 28 and the lighting window unit 38b. Should droplets move to pass the inclined guide surface 48 to reach the lighting window unit 38b, the droplets can be drawn into the distal channel opening 31, and removed easily by operation of suction.

In the above embodiment, the annular projection 45 and the inclined guide surface 48 are formed together with the end cap 36. However, the annular projection 45 can be formed with an element different from the end cap 36, for example, with the lens barrel 42. The inclined guide surface 48 can be formed with the lens barrel 42.

In the above embodiment, the endoscope 10 is a type having the CCD image sensor. However, the endoscope 10 of the invention can be a type in which an optical image guide device is used for transmitting image light.

In the above embodiment, the water conduit 26b and the gas conduit 26a are formed through the elongated tube 16. However, a single flow conduit can be formed through the elongated tube 16. A water conduit and a gas conduit can be provided upstream of the fluid supply button 23, which can connect a selected one of the water conduit and the gas conduit with the flow conduit by changeover.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope having an elongated tube (16) for entry in a body cavity, comprising:
a tip device (16a), disposed at a distal end of said elongated tube, and having a flat end surface (36c) extending perpendicularly to an axial direction of said elongated tube;
a viewing window unit (37), disposed to project from said end surface, for imaging of an object in said body cavity;
a nozzle device (28), disposed on said end surface, for ejecting fluid (49, 50) toward said viewing window unit;
a lighting window unit (38a, 38b), formed with said end surface, disposed in an ejection path (59, 60) of said nozzle device, for applying light to said object;
a suction opening (31), formed in said end surface, for suction of fluid from said body cavity;
a tapered surface (46), formed around said viewing window unit, and inclined toward said end surface;
an inclined guide surface (48), formed to extend from said tapered surface with an inclination between said viewing window unit (37) and said suction opening (31), for guiding said fluid,
**characterized by**
the inclined guide surface (48) being formed between the lighting window unit and the nozzle device and on the straight line passing the centers of the viewing window unit (37) and the suction opening (31), and by further comprising a concave side surface (48a) formed laterally with said inclined guide surface (48), opposed to said nozzle device (28), and curved concavely, the concave side surface (48a) being inclined in a tapered manner with respect to the flat end surface (36c).

2. An endoscope as defined in claim 1, **characterized in that** said nozzle device selectively ejects liquid and gas;
said ejection path of said gas is laterally larger than said ejection path of said liquid;
said viewing window unit is disposed inside said ejection path of said liquid, and said lighting window unit is disposed inside said ejection path of said gas.

3. An endoscope as defined in claim 1 or 2, **characterized in that** said lighting window unit is constituted by first and second lighting window units;
said inclined guide surface is disposed between said first lighting window unit and said nozzle device.

4. An endoscope as defined in claim 3, **characterized in that** said first lighting window unit is disposed farther from said nozzle device than said second lighting window unit, and nearer to said suction opening than said second lighting window unit.

5. An endoscope as defined in claim 3 or 4, **characterized in that** said inclined guide surface is disposed between said first and second lighting window units.

6. An endoscope as defined in any one of claims 1 to 5, **characterized in that** said tip device includes a head shell and an end cap mounted on a distal end of said head shell;
said inclined guide surface is formed with said end cap.

7. An endoscope as defined in any one of claims 1 to 6, **characterized in that** said suction opening is disposed close to said nozzle device.

8. An endoscope as defined in any one of claims 1 to 7, **characterized in that** said lighting window unit is disposed close to said tapered surface.

9. An endoscope as defined in any one of claims 1 to 8, **characterized in that** said suction opening is a distal channel opening for an instrument.

10. An endoscope as defined in any one of claims 1 to 9, **characterized in that** said viewing window unit protrudes from said end surface at a height equal to or more than 0.1 mm and equal to or less than 0.3 mm.

11. An endoscope as defined in any one of claims 1 to 10, **characterized in that** said lighting window unit is constituted by first and second lighting window units;
said suction opening is disposed between said nozzle device and said first lighting window unit, and said nozzle device is disposed between said second lighting window unit and said suction opening.

12. An endoscope as defined in any one of claims 1 to 11, **characterized in that** said nozzle device includes:
a nozzle sleeve, incorporated in said tip device, for flow of said fluid in said axial direction;
a nozzle opening, formed in a sleeve end of said nozzle sleeve to open in a direction crosswise to said axial direction, for ejecting said fluid from said nozzle sleeve on said end surface.

13. An endoscope as defined in any one of claims 1 to 12, **characterized in** further comprising:
a liquid conduit, provided through said elongated tube, for supplying said nozzle device with liquid;
a gas conduit, provided through said elongated tube, for supplying said nozzle device with gas;
a valve device for changing over said liquid conduit and said gas conduit, so as to change over said liquid and said gas to be ejected through said nozzle device.

## Patentansprüche

1. Endoskop mit einem länglichen Schlauch (16) für den Eintritt in einen Körperhohlraum, umfassend:
eine Endeinrichtung (16a), die an einem distalen Ende des länglichen Schlauchs angeordnet ist und eine flache Stirnfläche (36c) aufweist, die sich rechtwinklig zu einer axialen Richtung des länglichen Schlauchs erstreckt;
eine Betrachtungsfenstereinheit (37), angeordnet derart, dass sie von der Stirnfläche wegsteht, um ein Objekt in dem Körperhohlraum abzubilden;
eine Düseneinrichtung (28), die an der Stirnfläche angeordnet ist, um Fluid (49, 50) in Richtung der Betrachtungsfenstereinheit auszustoßen;
ein Beleuchtungsfenstereinheit (38a, 38b), die an der Stirnfläche ausgebildet ist und in einem Ausstoßweg (59, 60) der Düseneinrichtung gelegen ist, um Licht auf das Objekt aufzubringen;
eine Saugöffnung (31), die in der Stirnfläche ausgebildet ist, um Fluid aus dem Körperhohlraum anzusaugen;
eine um die Betrachtungsfenstereinheit herum ausgebildete und in Richtung der Stirnfläche geneigte verjüngte Fläche (46);
eine geneigte Führungsfläche (48), ausgebildet zur Erstreckung von der verjüngten Fläche mit einer Neigung zwischen der Betrachtungsfenstereinheit (37) und der Saugöffnung (31), um das Fluid zu leiten,
**dadurch gekennzeichnet, dass** die geneigte Führungsfläche (48) zwischen der Beleuchtungsfenstereinheit und der Düseneinrichtung und auf der geraden Linie, die durch die Mitten der Betrachtungsfenstereinheit (37) und der Saugöffnung (31) verläuft, ausgebildet ist, und dass weiterhin eine konkave Seitenfläche (48a) vorgesehen ist, die seitlich an der geneigten Führungsfläche (48) gegenüber der Düseneinrichtung (28) ausgeformt und konkav gekrümmt ist, wobei die konkave Seitenfläche (48a) in sich verjüngender Weise bezüglich der flachen Stirnfläche (36c) geneigt ist.

2. Endoskop nach Anspruch 1, **dadurch gekennzeichnet, dass** die Düseneinrichtung selektiv eine Flüssigkeit und Gas ausstößt;
der Ausstoßweg des Gases seitlich größer ist als der Ausstoßweg der Flüssigkeit;
wobei die Betrachtungsfenstereinheit sich im Inneren des Ausstoßwegs der Flüssigkeit befindet, und die Beleuchtungsfenstereinheit sich im Inneren des Ausstoßwegs des Gases befindet.

3. Endoskop nach Anspruch 1 oder 2, **dadurch gekennzeichnet, dass** die Beleuchtungsfenstereinheit durch eine erste und eine zweite Beleuchtungsfenstereinheit gebildet ist;
wobei die geneigte Führungsfläche sich zwischen der ersten Beleuchtungsfenstereinheit und der Düseneinrichtung befindet.

4. Endoskop nach Anspruch 3, **dadurch gekennzeichnet, dass** die erste Beleuchtungsfenstereinheit weiter von der Düseneinrichtung entfernt ist als die zweite Beleuchtungsfenstereinheit, und näher an der Ansaugöffnung liegt als die zweite Beleuchtungsfenstereinheit.

5. Endoskop nach Anspruch 3 oder 4, **dadurch gekennzeichnet, dass** die geneigte Führungsfläche sich zwischen der ersten und der zweiten Beleuchtungsfenstereinheit befindet.

6. Endoskop nach einem der Ansprüche 1 bis 5, **dadurch gekennzeichnet, dass** die Endeinrichtung eine Kopfhülse und eine Endkappe an einem distalen Ende der Kopfhülse enthält;
wobei die geneigte Führungsfläche an der Endkappe ausgebildet ist.

7. Endoskop nach einem der Ansprüche 1 bis 6, **dadurch gekennzeichnet, dass** die Saugöffnung nahe der Düseneinrichtung gelegen ist.

8. Endoskop nach einem der Ansprüche 1 bis 7, **dadurch gekennzeichnet, dass** die Beleuchtungsfenstereinheit sich in der Nähe der verjüngten Fläche befindet.

9. Endoskop nach einem der Ansprüche 1 bis 8, **dadurch gekennzeichnet, dass** die Saugöffnung eine distale Kanalöffnung für ein Instrument ist.

10. Endoskop nach einem der Ansprüche 1 bis 9, **dadurch gekennzeichnet, dass** die Betrachtungsfenstereinheit von der Stirnfläche in einer Höhe vorsteht, die gleich oder größer als 0,1 mm und gleich oder kleiner 0,3 mm ist.

11. Endoskop nach einem der Ansprüche 1 bis 10, **dadurch gekennzeichnet, dass** die Beleuchtungsfenstereinheit durch eine erste und eine zweite Beleuchtungsfenstereinheit gebildet ist;
wobei die Saugöffnung sich zwischen der Düseneinrichtung und der ersten Beleuchtungsfenstereinheit befindet, und die Düseneinrichtung sich zwischen der zweiten Beleuchtungsfenstereinheit und der Saugöffnung befindet.

12. Endoskop nach einem der Ansprüche 1 bis 11, **dadurch gekennzeichnet, dass** die Düseneinrichtung enthält:
eine Düsenhülse, die in die Endeinrichtung inkorporiert ist, um das Fluid in axialer Richtung strömen zu lassen;
eine Düsenöffnung, gebildet in einem Hülsenende der Düsenhülse zum Öffnen in einer Richtung quer zu der axialen Richtung, um das Fluid von der Düsenhülse an der Stirnfläche auszustoßen.

13. Endoskop nach einem der Ansprüche 1 bis 12, weiterhin umfassen:
eine Flüssigkeitsleitung, die durch den länglichen Schlauch verläuft und dazu dient, der Düseneinrichtung Flüssigkeit zuzuspeisen;
eine Gasleitung, die durch den länglichen Schlauch führt und dazu dient, der Düseneinrichtung Gas zuzuspeisen;
eine Ventileinrichtung zum Umschalten zwischen der Flüssigkeitsleitung und der Gasleitung, um dadurch umzuschalten zwischen dem Ausstoßen von Flüssigkeit und dem Ausstoßen von Gas über die Düseneinrichtung.

## Revendications

1. Endoscope présentant un tube oblong (16) pour l'entrée dans une cavité corporelle, comprenant :
un dispositif d'embout (16a), disposé sur une extrémité distale dudit tube oblong, et présentant une surface d'extrémité plate (36c) s'étendant perpendiculairement à une direction axiale dudit tube oblong ;
une unité de fenêtre de visualisation (37), disposée pour faire saillie à partir de ladite surface d'extrémité, pour imager un objet dans ladite cavité corporelle ;
un dispositif de buse (28), disposé sur ladite surface d'extrémité, pour éjecter un fluide (49, 50) vers ladite unité de fenêtre de visualisation ;
une unité de fenêtre d'éclairage (38a, 38b), formée avec ladite surface d'extrémité, disposée dans un trajet d'éjection (59, 60) dudit dispositif de buse, pour appliquer une lumière audit objet ;
une ouverture d'aspiration (31), formée dans ladite surface d'extrémité, pour l'aspiration de fluide à partir de ladite cavité corporelle ;
une surface présentant un amincissement progressif (46), formée autour de ladite unité de fenêtre de visualisation, et inclinée vers ladite surface d'extrémité ;
une surface de guidage inclinée (48), formée pour s'étendre à partir de ladite surface présentant un amincissement progressif avec une inclinaison entre ladite unité de fenêtre de visualisation (37) et ladite ouverture d'aspiration (31), pour guider ledit fluide,
**caractérisé par**
la surface de guidage inclinée (48) étant formée entre l'unité de fenêtre d'éclairage et le dispositif de buse et sur la ligne droite passant par les centres de l'unité de fenêtre de visualisation (37) et l'ouverture d'aspiration (31), et
par le fait qu'il comprend en outre une surface latérale concave (48a) formée latéralement avec ladite surface de guidage inclinée (48), face au dispositif de buse (28), et incurvée de manière concave, la surface latérale concave (48a) étant inclinée d'une manière présentant un amincissement progressif par rapport à la surface d'extrémité plate (36c).

2. Endoscope selon la revendication 1, **caractérisé en ce que** ledit dispositif de buse éjecte de manière sélective du liquide et du gaz ;
ledit trajet d'éjection dudit gaz est latéralement plus grand que ledit trajet d'éjection dudit liquide, et
ladite unité de fenêtre de visualisation est disposée dans ledit trajet d'éjection dudit liquide, et ladite unité de fenêtre d'éclairage est disposée dans ledit trajet d'éjection dudit gaz.

3. Endoscope selon la revendication 1 ou 2, **caractérisé en ce que** ladite unité de fenêtre d'éclairage est constituée de première et seconde unités de fenêtre d'éclairage, et
ladite surface de guidage inclinée est disposée entre ladite première unité de fenêtre d'éclairage et ledit dispositif de buse.

4. Endoscope selon la revendication 3, **caractérisé en ce que** ladite unité de fenêtre d'éclairage est disposée plus loin dudit dispositif de buse que ladite seconde unité de fenêtre d'éclairage, et plus près de ladite ouverture d'aspiration que ladite seconde unité de fenêtre d'éclairage.

5. Endoscope selon la revendication 3 ou 4, **caractérisé en ce que** ladite surface de guidage inclinée est disposée entre lesdites première et seconde unités de fenêtre d'éclairage.

6. Endoscope selon l'une quelconque des revendications 1 à 5, **caractérisé en ce que** le dispositif d'embout inclut une coque de tête et un chapeau d'extrémité monté sur une extrémité distale de ladite coque de tête, et
ladite surface de guidage inclinée est formée avec ledit chapeau d'extrémité.

7. Endoscope selon l'une quelconque des revendications 1 à 6, **caractérisé en ce que** ladite ouverture d'aspiration est disposée près dudit dispositif de buse.

8. Endoscope selon l'une quelconque des revendications 1 à 7, **caractérisé en ce que** ladite unité de fenêtre d'éclairage est disposée près de ladite surface présentant un amincissement progressif.

9. Endoscope selon l'une quelconque des revendications 1 à 8, **caractérisé en ce que** ladite ouverture d'aspiration est une ouverture de canal distale pour un instrument.

10. Endoscope selon l'une quelconque des revendications 1 à 9, **caractérisé en ce que** ladite unité de fenêtre de visualisation fait saillie à partir de ladite surface d'extrémité à une hauteur supérieure ou égale à 0,1 mm et inférieure ou égale à 0,3 mm.

11. Endoscope selon l'une quelconque des revendications 1 à 10, **caractérisé en ce que** ladite unité de fenêtre d'éclairage est constituée de première et seconde unités de fenêtre d'éclairage, et
ladite ouverture d'aspiration est disposée entre ledit dispositif de buse et ladite première unité de fenêtre d'éclairage, et ledit dispositif de buse est disposé entre ladite seconde unité de fenêtre d'éclairage et ladite ouverture d'aspiration.

12. Endoscope selon l'une quelconque des revendications 1 à 11, **caractérisé en ce que** ledit dispositif de buse inclut :
un manchon de buse, intégré dans ledit dispositif d'embout, pour l'écoulement dudit fluide dans ladite direction axiale ;
une ouverture de buse, formée dans une extrémité de manchon dudit manchon de buse pour s'ouvrir dans une direction en travers par rapport à ladite direction axiale, pour éjecter ledit fluide dudit manchon de buse sur ladite surface d'extrémité.

13. Endoscope selon l'une quelconque des revendications 1 à 12, **caractérisé en ce qu'**il comprend en outre :
une conduite de liquide, prévue à travers ledit tube oblong, pour alimenter ledit dispositif de buse en liquide ;
une conduite de gaz, prévue à travers ledit tube oblong, pour alimenter ledit dispositif de buse en gaz ;
un dispositif de soupape pour permuter entre ladite conduite de liquide et ladite conduite de gaz, de manière à permuter ledit liquide et ledit gaz à éjecter à travers ledit dispositif de buse.
